# EUROPEAN PATENT SPECIFICATION

(11) **EP 3 142 678 B1**
(45) Date of publication and mention of the grant of the patent: **11.03.2020**
(21) Application number: 15731396.6
(22) Date of filing: 11.05.2015
(51) Int. Cl.: A61K 36/8965, A61K 36/899, A61K 31/198, A61K 31/353, A61K 36/185, A61K 36/28, A61K 36/288, A61K 36/45, A61K 36/53, A61P 13/02

(54) **USE OF CRANBERRY PROANTHOCYANIDINS WITH HERBAL COMPONENTS FOR TREATING AND PREVENTING URINARY TRACT INFECTIONS**
VERWENDUNG VON KRANBEEREN-PROANTHOCYANIDINEN MIT PFLANZLICHEN KOMPONENTEN ZUR BEHANDLUNG UND VERHINDERUNG VON HARNWEGSINFEKTIONEN
UTILISATION DE PROANTHOCYANIDINES DE CANNEBERGE AVEC DES CONSTITUANTS À BASE D'HERBES POUR LE TRAITEMENT ET LA PRÉVENTION DES INFECTIONS DES VOIES URINAIRES

(30) Priority: 12.05.2014 EP 14167884
(43) Date of publication of application: 22.03.2017
(73) Proprietor: Specchiasol S.r.l., 37012 Bussolengo (VR) (IT)
(72) Inventor: SCIALPI, Antonio, 37012 Bussolengo (VR) (IT)
(74) Representative: Lisa, Elisabetta
(86) International application number: PCT/IB2015/053454
(87) International publication number: WO 2015/173715

(56) References cited:
- WO-A1-96/30033
- WO-A1-2011/088420
- WO-A1-2013/182702
- US-A1- 2008 213 401
- Specchiasol: "Specchiasol - NoCist Intensive", Facebook, 16 June 2014 (2014-06-16), XP055202412, Retrieved from the Internet: URL:https://www.facebook.com/specchiasol19 73/photos/pb.337173446319035.-2207520000.1 436948980./704066649629711/?type=1&theater [retrieved on 2015-07-15]
- Specchiasol: "Specchiasol - NoCist Prevent", Facebook, 12 June 2014 (2014-06-12), XP055202407, Retrieved from the Internet: URL:https://www.facebook.com/specchiasol19 73/photos/a.337715379598175.68918.33717344 6319035/701779029858473/?type=1&theater [retrieved on 2015-07-15]
- Rosari Kingston: "The Irish Medical Herbalist: Cystostop Rapid", The Irish Medical Herbalist, 16 June 2013 (2013-06-16), XP055202718, Retrieved from the Internet: URL:http://irishmedicalherbalist.blogspot. nl/2013/06/i-recently-posted-results-of-re search.html [retrieved on 2015-07-16]
- Specchiasol: "Nocist Intensive | Specchiasol", Specchiasol Natura Scienza Salute, 15 July 2015 (2015-07-15), XP055202419, Retrieved from the Internet: URL:http://www.specchiasol.it/it/prodotto/ fitoterapia/benessere-vie-urinarie/nocist- intensive [retrieved on 2015-07-15]
- SPECCHIASOL: "Nocist Prevent | Specchiasol", Specchiasol Natura Scienza Salute, 15 July 2015 (2015-07-15), XP055202415, Retrieved from the Internet: URL:http://www.specchiasol.it/it/prodotto/ fitoterapia/benessere-vie-urinarie/nocist- prevent [retrieved on 2015-07-15]
- DATABASE WPI Week 201420 Thomson Scientific, London, GB; AN 2014-E07014 & CN 103 479 833 A (UNIV SICHUAN WEST CHINA HOSPITAL) 1 January 2014 (2014-01-01)
- A Marchese: "Effect of fosfomycin alone and in combination with N-acetylcysteine on E. coli biofilms", International Journal of Antimicrobial Agents, vol. 22, 1 October 2003 (2003-10-01), pages 95-100, XP55024665, ISSN: 0924-8579, DOI: 10.1016/S0924-8579(03)00232-2

## Description

The present invention refers to new food supplements with innovative herbal component, suitable to be used against the common urinary tract infections.

As known, the urinary tract infections (UTI's) are caused by microbial infections due for the most part to the phenomenon infectious type ascending (rectum - vagino - urethral) of the bacterium named Escherichia coli that colonize the mucosa of the bladder causing the characteristic inflammation often disabling. Pharmacological treatment of the infection involves the use of specific antibiotics for urinary tract (trimethoprim-sulfamethoxazole, nitrofurantoin, fosfomycin, ciprofloxacin or levofloxacin). This disease is known for its characteristic form of recurrent acute phases, in practice it happens that after treating the acute phase with specific antibiotics after an unspecified period of time the infection appears again expounding all the typical virulence.

Recurrent cystitis is due to the ability of the bacterium Escherichia coli to evade the action of antibiotics, the body's immune responses in protecting a structure type called mucosal biofilms . Effective action against recurrent cystitis should always keep this in mind and to act even against biofilms of Escherichia coli, because only in this way you can try to end the infection cyclical feature.

Recent experimental results have confirmed the effectiveness of N-Acetylcysteine (NAC) in the disintegration and reduction in the number of viable forms of bacteria present in biofilms of Escherichia coli. These studies have been highlighted the effectiveness of two well-known antibiotics, and Thiamphenicol Fosfomycin trometamol, respectively used in respiratory infections and urinary tract infections uncomplicated. Particularly interesting is the new observation about the activity in association of N-Acetylcysteine and individual antibiotics, which was synergistic, opening up important new therapeutic perspectives in infectious diseases, chronic urinary tract infections caused by microorganisms forming biofilms, which, as is well known, it is almost impossible to eradicate with conventional antibiotic therapies. Many other studies confirm such effect: Four slime-producing uropathogenic Escherichia coli strains were used to investigate the activity of fosfomycin and N-acetylcysteine (NAC) against biofilms developed on 96-well polystyrene tissue culture plates. Biofilms aged, respectively, 5 (initial) and 48 h (mature) and two fosfomycin concentrations (128 and 2000 mg/l) were used. The effect of various levels (0.007-8 mg/ml) of NAC alone and in combination with fosfomycin on the formation or disruption of biofilms was assessed. Following exposure to the drugs, the percentage of residual slime relative to the control, ranged from 62.5-100 to 26.2-64.1% in the presence of 0.007 and 8 mg/ml of NAC. After treatment of pre-formed biofilms with NAC at the highest concentrations used, the remaining exopolysaccharide matrix was reduced to 25-68% of the amount found with the untreated control. Exposure to fosfomycin at 2000 mg/l reduced biofilms 40-57 and 41-49% for the initial and mature forms, respectively. Fosfomycin was more active at 2000 mg/l combined with NAC 2 mg/ml. Under these conditions initial and mature biofilms were reduced 66-80 and 60-73%, respectively. NAC, when used in combination, enhanced fosfomycin bactericidal activity producing a 99-99.9% reduction in viable cells. Fosfomycin and NAC at concentrations achievable in urine displayed a synergistic effect promoting both the formation of biofilms and reduction of sessile cell viability. The guidelines of the European Association of Urology suggest officially to intervene in these cases with alternative therapies to antibiotics in order to prevent the development of resistant strains and not to take chemotherapic antibiotics to patients for long periods. Among the alternative therapies the use of Cranberry is clearly recommended. Many official studies demonstrate that Cranberries extracts alone or together with other actives substancies, like for example D-mannose, are effective in case of UTI's also because they can be taken from the patience for long periods without side effects. For example, the food supplement known under the trade mark Cystostop Rapid™ by Neopharm comprises D-mannose, dry extract of birch leaves, dry extract of American cranberry and Proanthocyanidin 2A, and it is useful for the treatment of uncomplicated cystitis. Cranberries extract and D-mannose are not antibiotics, they work by preventing that bacteria can adhere to the bladder wall, and therefore, not being adherent to the bladder wall, are excreted with the urine flow. The therapeutic activity is due to the presence in the juice or extract of particular molecules defined polymeric proanthocyanidins (PACS). Many of such studies demonstrate therapeutic efficacy, but many others do not show an effectiveness of treatments. The cause of this lack of homogeneity in the results is to be found in the quality and quantity of proanthocyanidins in preparations tested. In fact, different titration methods show very different results in the amount of proanthocyanidins which can even be greater than 500 percent one respect to the other. Also the quality of proanthocyanidins detected can be deformed as to the morphological nature of PACS, polymers with different polymeric length can be detected , and this is reflected in a different rate of absorption, which will be faster for the smaller polymers chains. The absorption rate of the molecules at the intestinal level is a function of their size; shorter molecules are absorbed faster than long molecules. International scientific literature shows a good therapeutic activity of proanthocyanidins of the Cranberry, this activity is closely linked to the titration of proanthocyanidins that reflects the morphological type of the same. Cranberries exert a dose-dependent inhibition of the adherence of E. coli fimbriae to uroepithelial cells. This was demonstrated in vitro but also ex vivo in vitro with urine from cranberry consumers. The active principle has not been identified in detail but type-A proanthocyanidins (PAC) play an important role in the mechanism of action. Since the three species, American cranberry (Vaccinium macrocarpon), European cranberry (Vaccinium oxycoccus) and/or lingonberry (Vaccinium vitis-idaea), have different patterns of type-A PACs, results from one species cannot be transferred to the others. It seems likely that most of the studies with monopreparations from V. macrocarpon were underdosed. Whereas photometric PAC quantification may overestimate the true content on co-active compounds, reversed phase high-performance liquid chromatograpy may underestimate them. Recent studies with PAC doses in the upper range (DMAC method) or declared type-A PAC content in the daily dose reveal a dose-dependent trend of clinical effectiveness, however, with a possible ceiling effect. In order to clarify this, future three-arm studies should investigate Vaccinium preparations with higher type-A PAC doses than previously used.

The Applicant analyzed two popular European vitis-idaea products, a mother juice and a proprietary extract.

Both preparations may be appropriate to confirm the Vaccinium urinary tract infection-preventive effect beyond doubt. Positive clinical studies were the ones where extracts of Cranberry contained at least 36mg of PACS titrated with the validated method of analysis and sensitive called BL - DMAC. This improved DMAC method provides a simple, robust and relatively specific spectrophotometric assay for total PACs in cranberry samples using commercially available procyanidin A2 dimer as a standard. DMAC is most useful within a given type of food such as cranberries, but may not be appropriate for comparing concentrations across different food types, particularly in those cases where large differences exist among the relative amounts of each oligomer and polymer. A total of 19 different commercial cranberry products from American and European markets have been analyzed by different global phenolic methods and by UPLC-DAD-ESI-TQ MS. In addition, in vitro antioxidant capacity and uropathogenic bacterial antiadhesion activity tests have been performed. Results revealed that products found in the market widely differed in their phenolic content and distribution, including products completely devoid of flavan-3-ols to highly purified ones, either in A-type proanthocyanidins (PACs) or in anthocyanins. The product presentation form and polyphenolic profile widely affected the antiadhesion activity, ranging from a negative (nulel) effect to a MIC = 0.5 mg/mL for cranberry powders and a MIC=112 mg/mL for gel capsule samples. Only 4 of 19 products would provide the recommended dose of intake of 36 mg totalPACs/day. Of most importance it was the fact that this dose would actually provide as low as 0.00 and up to 205 µg/g of procyanidin A2, indicating the lack of product standardization and incongruence between global and individual compound analysis.

The present invention relates to an association comprising ProAnthoCyanidins (PACs) from cranberry extracts, N-Acetyl Cysteine (NAC) and diuretic herbal extracts, wherein the ProAnthoCyanidins comprise dimers, trimers and tetramers.

The intestinal absorbtion of molecules of small size is faster than that in which the molecules are large, and it is important to consider that molecules that are too large can not even be absorbed and therefore will not be present in the urinary tract where they carry out their work.

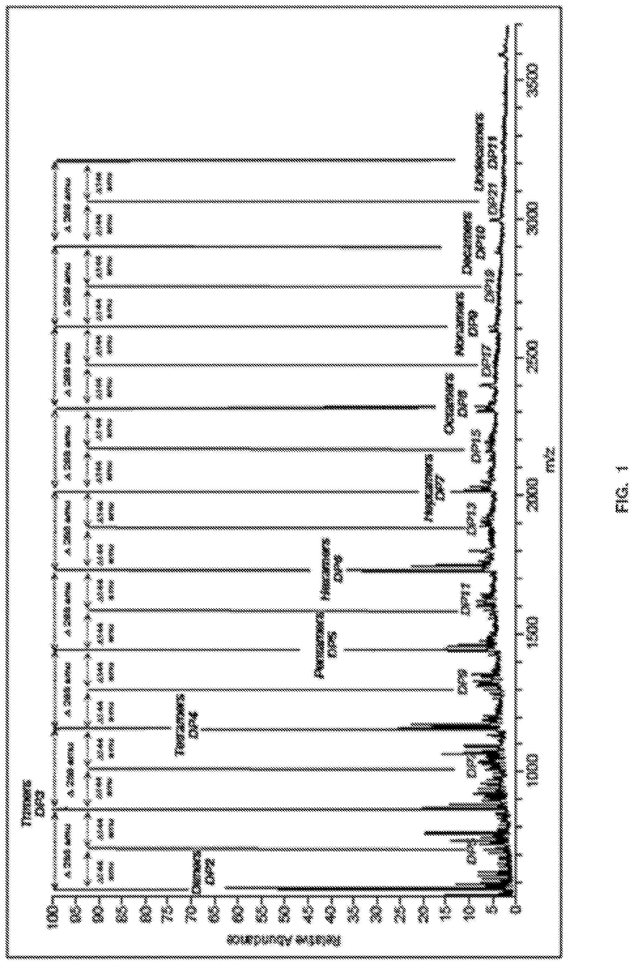

The determination of the quantities of the polymers is carried out thanks to HPLC analysis DAD MASSA. In the HPLC-ESI spectrum a series of intense signals differing by 288 amu is found (D288 amu in attached Fig.1), indicating the presence of the polyflavan-3-ol oligomers at different degree of polymerization (DP). Particularly, in this sample, signals up to DP11 (undecamers) are found. PACs give also signals assigned as doubly deprotonated molecular ions [M-2H]²⁻, in which case the different degree of polymerization is indicated by the D144 amu.

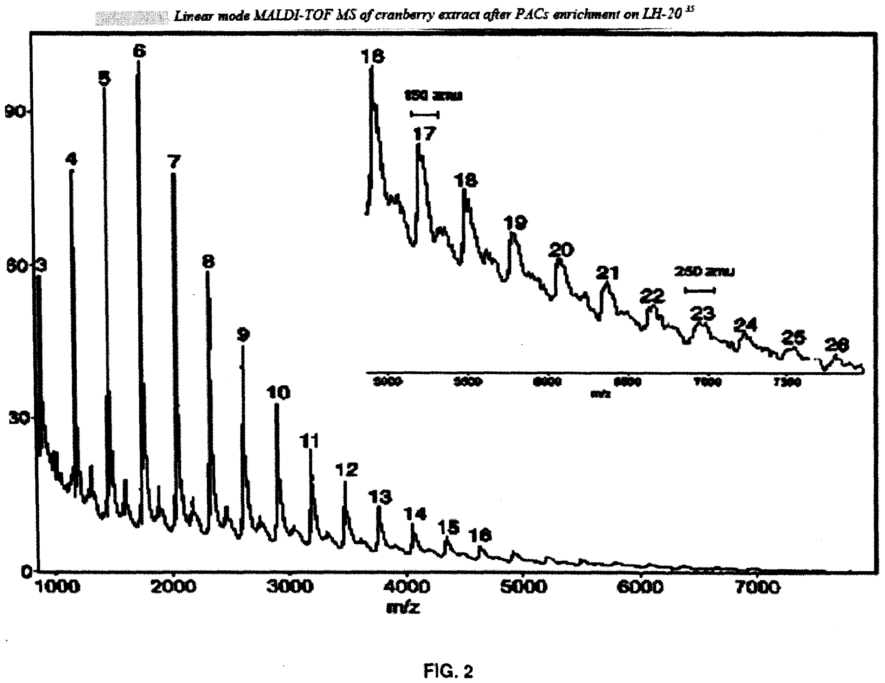

As double charged mass, signal up to 21-mers are detected, leading to MS characterization not far from those described for linear mode MALDI39, showed in attached Fig. 2 (MALDI-TOF spectrum - linear mode).

The Applicant, thanks to the application of these techniques, has been able to select different extracts of Cranberry in which the PACS are present in polymers from different length, this allows a specific use where one wishes to obtain a more rapid or a slower activity.

For these analyses the following tools have been used: HPLC-MSn: Cromatograph HPLC Varian HPLC 212 autosampler prostar 430, mass detector IT-500 ESI; HPLC-Fluorescence: cromatograph HPLC agilent 1260, diode array detector and fluorimeter.

The Applicant has therefore used for the first time chains of proanthocyanidins (PACS) of different length in order to better modulate the effects of the above :
a) short chains: fast rate of absorption and action.
b) medium to long chains: average rate of absorption and action.

With the use of short-chain polymers it is possible to realize products for the treatment of acute forms of cystitis while with the use of chain polymers.

Average long products are made for the treatment of relapsing forms of cystitis.

An example of an analysis of a dry extract of cranberry relatively to the length of the polymers is shown in the table below which relates to the relative percentages of each class of PAC in three types of extracts considered:

| | % of PACS SAMPLE 4225 | % of PACS SAMPLE 4223 | % of PACS SAMPLE 4224 |
|---|---|---|---|
| Dimers | 5.1 | 2.8 | 2.4 |
| Trimers | 3.7 | 1.075 | 0.85 |
| Tetramers | 5.2 | 0.9 | 0.6 |

The table further shows that different extracts of Cranberry contain different percentages of dimers and trimers, tetramers.

The invention is intended to cover associations of PACS, from 36 mg to 120 mg of PACS, where the lenght of the polimers chains are known.

The second aspect is related to the association of N-Acetyl Cysteine with extracts of PACS in different lengths of polymer chains.

According to the international scientific literature, N-Acetyl Cysteine is able to be effective against the biofilm of Escherichia coli by exposing the bacterium to the action of the extracts of Cranberry antibiotic used.

The third aspect is related to the combination of the first two aspects with the adding of phytotherapeutic extracts with diuretic-draining liquids activity, allowing a fast water exchange at the level of the bladder which then allows a faster and more effective removal of infectious agents from urinary tract.

Among the plants subject to aspect the Applicant indicates: Betula alba, Orthosiphon stamineus, Solidago virgaurea, Agropyron repens, Hieracium pilosella, Asparagus officinalis, Zea mays, Taraxacum officinalis.

The fourth aspect is related to the combination of the cited three associations with adding the molecule D-mannose, molecule useful for improving the yield of the other ingredients.

## Claims

1. An association comprising ProAnthoCyanidins (PACs) from Cranberry extracts, N-Acetyl Cysteine (NAC) and diuretic herbal extracts, wherein the ProAnthoCyanidins (PACs) comprise a mixture of dimers, trimers and tetramers.

2. An association according to claim 1, wherein the diuretic herbal extracts are selected from among *Hieracium pilosella, Betula alba, Solidago virgaurea.*

3. An association according to claim 1 or 2, further comprising d-Mannose.

4. An association according to any of the preceding claims for use as a medicament in the prevention and/or treatment of urinary tract infections (UTIs).

5. An association according to claim 4, wherein urinary tract infections (UTIs) is cystitis.

## Patentansprüche

1. Assoziation bestehend aus Proanthocyanidinen (PACS) aus Cranberry-Extrakten, N-Acetylcystein (NAC) und Extrakten von diuretischen Kräutern, wobei die Proanthocyanidine (PACS) eine Mischung aus Dimeren, Trimeren und Tetrameren enthalten.

2. Assoziation nach Anspruch 1, wobei die Extrakte von diuretischen Kräutern ausgewählt sind aus *Hieracium pilosella, Betula alba, Solidago virgaurea.*

3. Assoziation nach Anspruch 1 oder 2, weiter enthaltend d-Mannose.

4. Assoziation nach einem der vorhergehenden Ansprüche zur Verwendung als Medikament zur Vorbeugung und/oder Behandlung von Harnwegsinfektionen (HWI).

5. Assoziation nach Anspruch 4, wobei eine Harnwegsinfektion (UTI) eine Blasenentzündung ist.

## Revendications

1. Association comprenant des proanthocyanidines (PACS) à partir d'extraits de Cranberry, N-acétyl cystéine (NAC) et d'extraits d'herbes diurétiques, dans laquelle les proanthocyanidines (PACS) comprennent un mélange de dimères, trimères et tetramères.

2. Association selon la revendication 1, dans laquelle les extraits d'herbes diurétiques sont sélectionnés entre *Hieracium pilosella, Betula alba, Solidago virgaurea.*

3. Association selon la revendication 1 ou 2, comprenant aussi d-mannose.

4. Association selon l'une des revendications précédentes pour emploi comme un médicament dans la prévention et/ou le traitement des infections des voies urinaires (IVU).

5. Associations selon la revendication 4 dans laquelle une infection des voies urinaires (IVU) est cystite.
